# EUROPEAN PATENT SPECIFICATION

(11) **EP 2 566 546 B1**
(45) Date of publication and mention of the grant of the patent: **30.03.2016**
(21) Application number: 11720615.1
(22) Date of filing: 13.04.2011
(51) Int. Cl.: A61M 15/00

(54) **DRY POWDER INHALER MOUTHPIECE BUTTON**
TROCKENPULVERINHALATOR-MUNDSTÜCK
BOUTON D'EMBOUT BUCCAL POUR INHALATEUR À POUDRE SÈCHE

(30) Priority: 28.05.2010 TR 201004307; 20.04.2010 TR 201003091; 13.04.2010 TR 201002877
(43) Date of publication of application: 13.03.2013
(73) Proprietor: Sima Patent ve Lisanslama Hizmetleri Ltd.Sti., Esenler/Istanbul (TR)
(72) Inventor: BILGIC, Mahmut, D Blok 34220 Esenler-Istanbul (TR)
(86) International application number: PCT/TR2011/000085
(87) International publication number: WO 2011/129785

(56) References cited:
- US-A1- 2004 094 152
- US-A1- 2005 154 491
- US-A1- 2005 268 909
- US-A1- 2007 062 525
- US-A1- 2009 078 252

## Description

### Field of the Invention

The present invention relates to an inhaler which is appropriate for delivery of medicament in dry powder form used in respiratory diseases, particularly in asthma and chronic obstructive pulmonary disease (COPD). In addition, the present invention relates to an inhaler comprising a blister package which is appropriate for carrying the medicament in dry powder form and used to realize an effective inhalation.

### Description of the Prior Art

It is rather common to use inhalers for delivering medicaments utilized in the treatment and prophylaxis of respiratory diseases. Inhalation treatment is the most commonly preferred treatment method in these diseases as the inhalers provide ease of use; the medicaments have rapider onset of time resulting from local administration and they have fewer side effects. Various inhalers have been designed in order to provide effective and sufficient delivery of the medicaments used in the treatment of respiratory diseases, particularly in asthma and chronic obstructive pulmonary disease. These inhalers vary according to their operating mechanisms and the physical form of the medicament to be delivered.

In the inhalers used to deliver the medicaments in dry powder form, the medicament is carried in reservoirs, capsules or blisters packages. It is highly significant to deliver each dose to the patient with exact accuracy and preciseness since the required medicament dose in the inhalation is very low.

In general, one blister pocket containing medicament in dry powder form is opened in response to each actuation of the device in inhalers comprising blister packages. One blister pocket containing one dose of dry powder medicament is usually opened by peeling the blister package indexed upon the actuation of the device or piercing the blister pocket by the piercing means in the inhaler. The inhalers comprising peelable blister packs enable the sufficient amount of the dry powder medicament contained in the opened blister to be easily inhaled as the airflow enters the opened blister pocket more easily in the inhalers comprising peelable blister packs than the inhalers comprising pierceable blister packs. Therefore, the blister package should be indexed enough to enable the blister pocket to be opened completely so as to realize an effective inhalation in response to each actuation of the inhaler. However, it is quite difficult to enable the blister package to be indexed properly to the same extent in each actuation of the device so as to realize a safe inhalation in the inhaler comprising peelable blister packages. In the case that the blister package that is indexed upon the actuation of the device is indexed less than the required extent, the blister pocket may not be opened completely while more than one blister pocket may be opened in the case that the blister package is indexed more than the required extent. The fact that one blister pocket cannot be opened completely and an effective inhalation cannot be realized as the sufficient amount of the active agent comprised in the dry powder medicament cannot be delivered to the patient or more than the required amount of the active agent is delivered to the patient as one blister pockets are opened lead to dangerous consequences. Therefore, controlled dosing of the medicament in dry powder form cannot be achieved when the blister package is not indexed properly to the same extent in response to each actuation of the inhaler.

The inhalation device marketed under the trade mark Diskus® by GlaxoSmithKlein is one of the most well-known inhalers on the market. This device operates with a slide mechanism and a blister strip package in which the dry powder medicament is carried. However, this device needs to be improved in terms of specifications to enable the blister package to be properly indexed to the same extent in response to each actuation of the device.

The inventor has surprisingly found that the force of attraction imposed by the winding wheel on the lid sheet is balanced, and thus the blister package is properly indexed to the same extent in response to each actuation of the device in the case that each of the preferably polyoxymethylene resilient wings of the winding wheel, on which the lid sheet of the blister package peeled upon the actuation of the inhaler is coiled, is composed of three parts in the inhaler comprising peelable blister package.

To this respect, the present invention relates to an inhaler comprising peelable blister package appropriate for delivering dry powder medicament which enables the blister package to be indexed properly to the same extent in response to each actuation of the inhaler.

Documents US 2005/0154491 A1 and US 2009/0078252 A1 both describe powder inhalers with winding wheels similar to that of the invention.

### Summary of the Invention

The scope of the invention is as defined by the appended claims.

An inhaler suitable for delivery of the medicament in dry powder form according to the present invention comprising;
- a blister package composed of a plurality of blister pockets each of which comprises medicament in dry powder form and which are spaced at equal intervals,
- a mouthpiece enabling the patient to inhale the medicament in dry powder form from the opened blister,
- a gear mechanism enabling the blister package to be indexed and the medicament in dry powder form to become ready for inhalation;
- the winding wheel which is a member of the gear mechanism and on which the lid sheet of the blister pack is coiled,
- a rotatable mouthpiece cover covering the mouthpiece;
- a housing situated between the upper housing member and the lower housing member in which the blister package and the gear mechanism are enclosed
- each of the resilient wings of the winding wheel extending from the center to the end of the winding wheel on which the lid sheet of the blister package that is indexed and peeled upon the actuation of the inhaler is coiled is comprised of 3 parts (A, B, C); and the average radius (R1) of the second part (B) of these parts composing each of the resilient wings extending from the center to the end of the winding wheel is in the range of 4,60 mm to 5,20 mm; and the radius (R2) of the piece of the third part (C) that curls through the end of the resilient wing is in the range of 0,9 mm to 1,70 mm.
- the mouthpiece cover (2) can solely be in two positions: - the mouthpiece (14) is completely covered and the device (1) is on standby mode when the mouthpiece cover (2) is in the first position, one dose of the medicament in dry powder form is ready for inhalation upon the actuation of the device (1) when the mouthpiece cover (2) is in the second position.

As each of the resilient wings extends from the center to the end of the winding wheel, the points of the resilient wings are also deemed to be the points of the winding wheel.

According to the present invention, the blister pocket is indexed enough to enable one blister pocket to be opened and it is peeled in the meanwhile in response to each actuation of the device. After the blister package is peeled, each layer is accumulated in a different compartment of the inhaler. The lid sheet of the blister package that is one of the peeled layers is tightly coiled on the resilient wings of the winding wheel which is one of the components of the gear mechanism. Thus, a force of attraction is imposed on the lid sheet, therefore on the blister package by the winding wheel. The diameter of the winding wheel increases as the thickness of the lid sheet coiling on the winding wheel while the blister package is indexed in response to each actuation of the inhaler increases, and thus the magnitude of the force of attraction imposed on the blister package by the winding wheel increases. This results in the blister package to be pulled more strongly and indexed to various extents in each actuation of the inhaler. In addition, the more the force of attraction imposed on the blister package by the winding wheel, the more probable it is that the blister package is broken off. The force of attraction imposed on the blister package by the winding wheel has to be stable for the blister package to be indexed properly to the same extent in each actuation of the inhaler. The inventor has surprisingly found that the force of attraction imposed on the blister package indexed upon the actuation of the device by the winding wheel can always remain stable in the case that each of the resilient wings of the winding wheel which are preferably made of polyoxymethylene plastics (generally identified as POM and it is also known as polyacetal or polyformaldehyde) are composed of 3 basic parts (A, B, C), and the average radius (R1) of the second part (B) of each resilient wing concatenated from the center to the end of the winding wheel is in the range of 4,60 mm to 5,20 mm; and the radius (R2) of the piece of the third part (C) that curls through the end of the resilient wing is in the range of 0,9 mm to 1,70 mm. Thus, the resilient wings of the winding wheel can stretch enough to balance the increasing force of attraction imposed on the blister package as the thickness of the lid sheet coiling on the winding wheel.

Each part of the resilient wings of the winding wheel, which is a component of the gear mechanism of the inhaler pertaining to the present invention, is preferably made of the same substance.

Thanks to the resilient wings preferably made of polyoxymethylene plastics which are used in the inhaler pertaining to the present invention and hold the specified sizes, the blister pocket is opened completely and controlled dosing of the dry powder medicament used in the treatment is achieved as the blister package is indexed properly to the same extent in response to each actuation of the inhaler. Furthermore, an effective inhalation is realized as delivery of sufficient amounts of the active agent to the patient is enabled during each inhalation.

The term "controlled dosing" used in this context refers to delivering approximately the same amount of active agent to the patient in each actuation of the inhaler.

The term "effective inhalation" used in this context refers to inhalation during which sufficient amount of active agent is inhaled.

According to the present invention, the inhaler is an easy-grip, manual device which is appropriate for delivering medicament in dry powder form.

The housing of the device pertaining to the present invention has been designed such that each component of the blister package and the gear mechanism which have a significant role in enabling the device to work properly is situated accurately and works harmoniously. To this end, the housing is divided into several compartments. The used portion and the unused portion of the blister package are accommodated in partitioned compartments in order to prevent the medicament in dry powder form remained in the opened blister pocket to spill on the other components of the housing. Furthermore, the housing also comprise the beak which enables the blister package to be peeled and the manifold through which the dry powder medicament in the open blister passes before reaching the mouthpiece during the inhalation. In addition, the housing can be in any appropriate shape while it is preferably elliptic or circular.

The upper and the lower housing members interlock with each other and enclose the housing in order to keep the housing and the gear mechanism fixed together. The mouthpiece cover hiding the mouthpiece is rotated by being slid on the upper and lower housing members. The grids on the surface of the lower and the upper housing members provide an effective actuation by preventing the slipping of the finger while rotating the mouthpiece cover. The upper and the lower housing members can be in any appropriate shape or size which provides ease of use.

The inhaler pertaining to the present invention is preferably actuated by the mouthpiece cover. Before each inhalation, both the mouthpiece is exposed and one dose of the medicament in dry powder form becomes ready for inhalation as one of the blister pockets is opened as a result of the mouthpiece cover's preferably being manually rotated along the path restricted by the engagement of the protrusions on the upper and the lower housing members. The rotational path on which the cover moves is restricted on both ends by the protrusions of the upper and the lower housing members. The constant-distance path that the protrusions of the upper and the lower housing members define results in the mouthpiece cover's being rotated by the same angle in response to the each actuation of the device.

The mouthpiece cover of the device is joined with the gear mechanism by the connection points. One end of the drive gear passes through the center of the lower housing member and tightly joins with the mouthpiece cover in one connection point while the other end passes through the center of the upper housing member and tightly joins with the mouthpiece cover in the other connection point. For each end of the drive gear to make a fixed connection between the connection points of the mouthpiece cover, one side cover is used for each end of the drive gear. The ends of the drive gear are carved for the ends of the side covers to be joined with each end of the drive gear from inside such that the ends of the side covers can tightly interlock with it. Inner faces of these carved parts in each end of the drive gear have a matching shape with the shapes of the ends of the side covers. These side covers that attach with the each end of the drive gear passes through the connection point and provide the mouthpiece cover to synchronize with the drive gear.

On each connection point of the mouthpiece cover, there is a stabilizing resilient cover. The extensions under the stabilizing resilient covers pass through the holes on the upper or the lower housing member according to the position of the connection point and provide the stabilizing resilient covers that they connect with to remain stable. The rotation of the mouthpiece cover is prevented from both sides as the pawl under each of the stabilizing resilient covers in both sides of the device interlocks with the mouthpiece cover. Before the inhalation, the resilient parts of each stabilizing resilient cover which are compatible with the shape of the fingers are pressed on for raising the pawls and releasing the mouthpiece cover in order to move the mouthpiece cover which actuates the device. Thus, the mouthpiece cover can easily be rotated when the resilient parts of each stabilizing resilient cover on both sides of the device which match with the shape of the fingers are pressed on. When the resilient parts of the stabilizing resilient covers are not pressed on, the pawls under the stabilizing resilient covers do not allow the mouthpiece cover to move in any circumstances.

The mouthpiece cover that actuates the device can solely be in two positions. For the gear mechanism that indexes the blister package to be triggered, the mouthpiece cover should be moved from the first position to the second position. When the mouthpiece cover is in the first position, it leans on the protruding part in one end of the rotational path. The mouthpiece is completely hidden when the mouthpiece cover is in the first position and the device is on standby mode. When the mouthpiece cover is in the second position, it leans on the protruding part on the other end of the rotational path and one dose of the medicament in dry powder form is prepared for inhalation upon the actuation of the device.

According to the present invention, each gear in the gear mechanism is directly or indirectly engages with each other. The constant-angle rotational movement of the mouthpiece cover is transmitted to the indexing ratchet wheel which engages with the drive gear in each actuation of the inhaler owing to the connection between the mouthpiece cover and the drive gear via the side covers. The indexing ratchet wheel that interlocks with the indexing wheel from inside thanks to its arms causes the indexing wheel to move 45°. Upon the rotation of the indexing wheel, the blister package is indexed and peeled by the beak in the housing. As the indexing wheel engages with the winding wheel gear and the pinion gear, these gears synchronize with the indexing wheel. Since the mechanism gear engages both with the winding wheel gear and the winding wheel, the rotation of the winding wheel gear causes the winding wheel to move and the lid sheet of the blister package is coiled on the winding wheel tightly. The pinion gear engages with the base gear while the small gear right under the base gear engages with the counter gear. The rotation of the indexing wheel is transmitted to the base gear via the pinion gear. As the small gear under the base gear synchronizes with the base gear, the rotation of the base gear rotates the counter gear.

According to the invention, the mechanism gear engaging with the winding wheel gear interlocks with the winding wheel. Therefore, the rotation of the winding wheel gear is transmitted to the winding wheel via the mechanism gear in response to each actuation of the device. Actuation of the device results in the blister package's being indexed and peeled. The lid sheet of the blister package which is one of the peeled layers is coiled on the winding wheel while the blister package is indexed. The winding wheel has resilient wings preferably made of polyoxymethylene plastics on which the lid sheet of the blister package is coiled. These resilient wings stretch as the thickness of the lid sheet of the blister package that coils on it increases, and therefore it balances the force of attraction imposed on the blister package. According to the invention, in order to provide the blister package to be indexed properly to the same extent in each actuation of the inhaler, the resilient wings of the winding wheel that are preferably made of polyoxymethylene plastics have to be composed of 3 different parts for the force of attraction imposed on the blister package to be stable all the time. The average radius (R1) of the second part (B) of each resilient wing concatenated from the center to the end of the winding wheel is in the range of 4,60 mm to 5,20 mm, preferably in the range of 4,75 mm to 5, 15 mm; and the radius (R2) of the piece of the third part (C) that curls through the end of the resilient wing is in the range of 0,9 mm to 1,70 mm, preferably in the range of 1,10 mm to 1, 50 mm. The length of the first part (A) of the resilient wing of the winding wheel from the center to the end approximately equals to the sum of the lengths of the second (B) and the third (C) parts of the resilient wing of the winding wheel (±3 mm). The length of the second part (B) of the winding wheel is maximum as much as the length of the third part (C) of the winding wheel.

According to the present invention, the indexing wheel is another component of the gear mechanism and it provides the blister package to be indexed properly and the opened blisters to be positioned accurately. The recesses of the indexing wheel match with the shape of the blister package and the blister pockets of the blister package are received in these recesses in sequence while the indexing wheel rotates. There are preferably 8 recesses on the indexing wheel. Therefore, the indexing wheel is supposed to rotate by the same angle in response to each actuation of the device for the opened blister pocket to be positioned accurately.

At least one stopper component is arranged in any suitable part of the device in order to provide the blister package to be precisely positioned. The stopper can be in any suitable shape to execute said task.

The counter gear in the device pertaining to the present invention displays the number of the unused blister pockets remained in the device. In response to the actuation of the device by the mouthpiece cover, the mouthpiece is uncovered, the blister package is indexed and one dose of the dry powder medicament is prepared for inhalation while the counter gear rotates as well. Thus, the movement of the mouthpiece cover leads to the mouthpiece cover to be uncovered; one dose of the dry powder medicament to be ready for inhalation after the blister pocket is opened as well as providing the counter gear to rotate and display the new value of the unused blister pockets remained.

On the counter gear, there exist numerals equal to the number of the blister pockets present in the device and they are spaced by equal angles. In a device comprising 60 doses, the angle between the numerals is approximately 5°. The counter gear rotates as a result of the reflection of the rotation of the indexing wheel via the pinion gear and the base gear. In response to the each actuation of the device, rotation of the indexing wheel by the same angle each time due to the accurate transmission of the movement of the mouthpiece to the gear mechanism via the drive gear results in the rotation of the counter wheel approximately by the same angle as well and each numeral on the counter wheel is clearly seen through the display aperture on the upper housing member. Therefore, the patient makes sure about the number of the unused blister pockets remained in the device.

The inhaler according to any of the proceeding claims, further comprising a blister package composed of a plurality of blister pockets each of which comprises medicament in dry powder form and which are spaced at equal intervals. The blister package carries the medicament in dry powder form in one-dose portions and it is preferably a blister strip and it is preferably peelable.

The blister pockets comprised in the blister package are spaced in equal intervals and each of them carries one dose of the medicament in dry powder form.

While the blister package is indexed on the indexing wheel, the beak on the housing peels the blister. Therefore, one dose dry powder medicament becomes ready for inhalation after the blister package is peeled to be opened in each actuation of the device.

The base sheet of the blister package on which the blister cavities are spaced is accumulated in the separated compartment of the housing. The lid sheet that provides impermeability of the blister package, on the other hand, is coiled on the winding wheel which is one of the components of the gear mechanism positioned in the other side of the housing.

The blister opened by the beak is situated immediately under the manifold. Upon the inhalation of the patient, the airflow that preferably enters the device through at least one air inlet on the upper housing member entrains the dry powder medicament in the opened blister pocket via the manifold to the mouthpiece and enables the delivery of said medicament to the patient. The air inlet on the upper housing member that allows the entry of air can be in any suitable shape and size that also enable external air to enter the device easily and at a convenient speed.

The air inlet that the external airflow passes through is preferably designed not to be close where the patient holds the device in order not to prevent the air flow. Furthermore, in order to deliver the required amount of the dry powder medicament in the opened blister to the patient, the air inlet has been designed such that it allows the entry of the airflow through the air inlet by a convenient angle.

One end of the manifold communicates with the opened blister while the other end communicates with the mouthpiece. On the end of the manifold that communicates with the blister, at least two apertures with four sub-apertures are situated. Upon the inhalation of the patient, some of the air flow which enters through the air inlet passes through one of these apertures; entrains the medicament in dry powder form through the aperture to the manifold. The medicament in dry powder form that is entrained to the manifold with the airflow is delivered to the patient via the mouthpiece. The manifold can be in any appropriate shape while its length is preferably at least 1 mm.

The mouthpiece is designed to fit the mouth for the patient to comfortably inhale the medicament in dry powder form. According to the shape of the device, the mouthpiece can be in any suitable shape or size as well as being fixed or movable. Furthermore, it can be attached or unattached to the upper and/or the lower cover.

Each component of the device pertaining to the present invention can be made of any suitable material while they are preferably be made of plastic. These plastics can preferably be chosen from a group comprising the types of styrene acrylonitrile, polyoxymethylene (acetale), acrylic-polymethylmetacrylate, cellulose acetate, polyetheretherketone, polyvinyl choloride, polyethylene, polypropylene, acrylonitrile butadiene styrene, polycarbonate, polyamide, polystyrene, polyurathane or fluoropolymer while it is preferably polyoxymethylene acetale. The plastic components can be manufactured by methods like injection molding. Moreover, each component of the device can be in any suitable color.

The lid and the base sheets constituting the blister package preferably consist of a plurality of layers. Each of these layers is preferably selected from a group comprising polymeric layers made of various polymeric substances, aluminum foil and preferably fluoropolymer film.

According to the present invention, the lid and base sheets composing the blister package are sealed very tightly by at least one of the methods comprising cold formed bonding, hot metal bonding, hot metal welding, radio frequency welding, laser welding or ultrasonic welding in order to provide impermeability, more preferably by cold formed bonding method. Since these cold formed bonding methods can be carried out at lower temperatures than hot sealing methods, they are the most appropriate methods to use in the case that the medicament carried in the blister is heat sensitive.

Fluoropolymer film is a polymeric film which is used in blister packs and provides excellent moisture barrier. This chemically inert polymeric film does not cause any change in the taste of the formulation when it is in contact with the dry powder formulation. In addition, it easily constitutes a layered structure with the other polymeric layers which are composed of various polymers. It is appropriate to be transacted with heat.

For preserving the stability of the dry powder formulation stored in the blister package, preferably at least one of the polymeric layers comprises at least one desiccant agent including silica gel, zeolite, alumina, bauxite, anhydrous calcium sulfate, activated carbon and clay which has the property of water absorption in order to decrease gas and moisture permeability of the layer.

According to the invention, the thickness of the aluminum foil in the lid and the base sheets of the blister package are preferably chosen to be in the range of 5 to 80 µm, more preferably in the range of 15 to 65 µm.

According to the invention, the polymeric layers in the lid and the base sheets of the blister pack are made of the same or different polymers. The thickness of these polymeric layers varies according to the type of the polymeric substance used and its properties while they are preferably in the range of 5-100 µm, more preferably in the range of 15-60 µm.

The polymers composing the polymeric layer are preferably selected from thermoplastics such as polyethylene, polypropylene, polystyrene, polyolefin, polyamide, polyvinyl chloride, polyurethane or synthetic polymers.

The blister pockets in the blister package can be in any appropriate shape. The plurality of blister pockets spaced at equal intervals on the base sheet of the blister package can be in the same or different shape, structure or volume.

The reference numbers of the drawings added to exemplify the present invention and the detailed description of the invention according to these drawings are given below but the scope of the invention should not be limited to these drawings.

### Brief Description of the Drawings

Figure 1 is a perspective view of an inhaler according to the inhaler described in the present invention;
Figure 2 is an exploded view of the inhaler pertaining to the invention;
Figure 2a is a perspective view of the winding wheel pertaining to the present invention,
Figure 3 is a perspective view of the blister pack for use with the inhaler pertaining to the invention;
Figures 4a and 4b are perspective views of the housing of the inhaler according to the invention;
Figures 5a and 5b are perspective views of upper and lower housing members of the inhaler according to the invention;
Figure 6a is a perspective view of the mouthpiece cover of the inhaler pertaining to the invention;
Figure 6b is an exploded view of the communication between the mouthpiece cover, the drive gear and the stabilizing resilient covers in the inhaler pertaining to the invention;
Figure 6c is a cross-sectional view of the communication between the mouthpiece cover, the drive gear and the stabilizing resilient covers in the inhaler pertaining to the invention;
Figure 6d is a cross-sectional view of the communication between the mouthpiece cover, the drive gear and the stabilizing resilient covers in the inhaler pertaining to the invention;
Figure 6e is an exploded view of the communication between the drive gear and the side covers in the inhaler pertaining to the invention;
Figure 6f is a cross-sectional view of the connection of the stabilizing resilient cover with the lower housing member in the inhaler pertaining to the invention;
Figures 7a-7c are cross-sectional views of the engagement of the gears composing the gear mechanism with each other in the inhaler pertaining to the present invention;
Figure 8 is a cross-sectional view of the blister package delaminating in course of operation of the inhaler pertaining to the present invention;
Figure 9 is a perspective view of the counter gear used in the inhaler pertaining to the present invention.

### Detailed Description of the Drawings

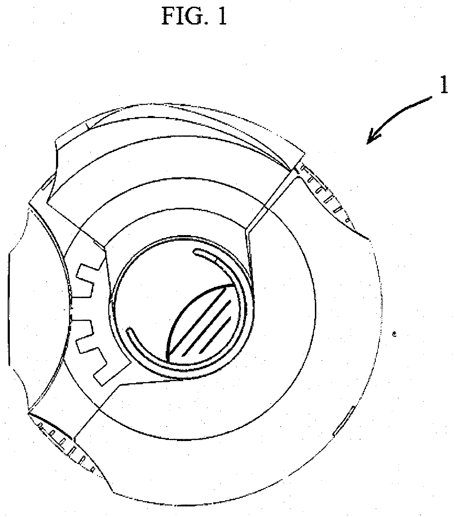

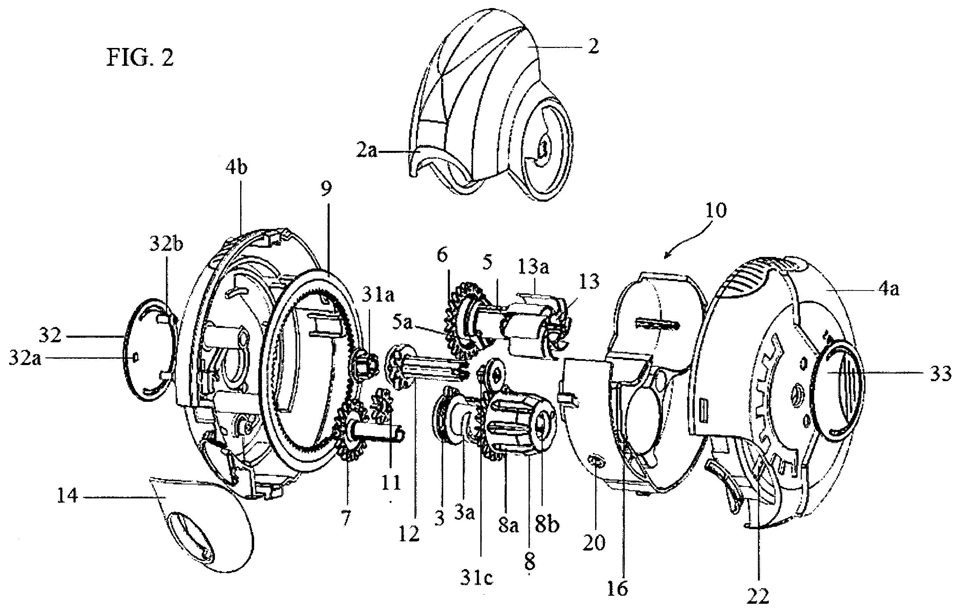

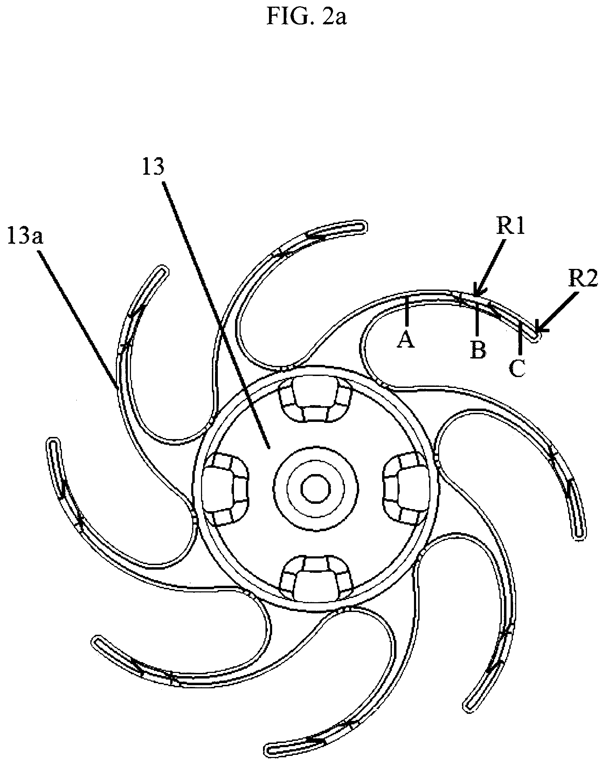

The inhaler (1) pertaining to the present invention comprises a gear mechanism situated in the housing (10) between the upper housing member (4a) and the lower housing member (4b) in order to enable the inhalation of the dry powder medicament carried in a blister package (15) as displayed in figures 1 and 2. Each component of the inhaler (1) is positioned at suitable spots on the housing (10) to guarantee their working properly and accurately.

The inhaler (1) pertaining to the present invention shown in figure 1 is ready for inhalation. In this case, the mouthpiece cover (2) is in the second position and the mouthpiece (14) is entirely exposed. The mouthpiece cover (2) has to be rotated by holding on the carved part (2a) on one end of the mouthpiece cover (2) in order to switch to the second position from the first position wherein the mouthpiece is completely covered. In this way, the mouthpiece (14) is completely exposed when the mouthpiece cover (2) is switched to the second position from the first position and the gear mechanism is triggered by the drive gear (12). The drive gear (12) precisely transmits the movement of the mouthpiece cover (2) to the indexing ratchet wheel (3).

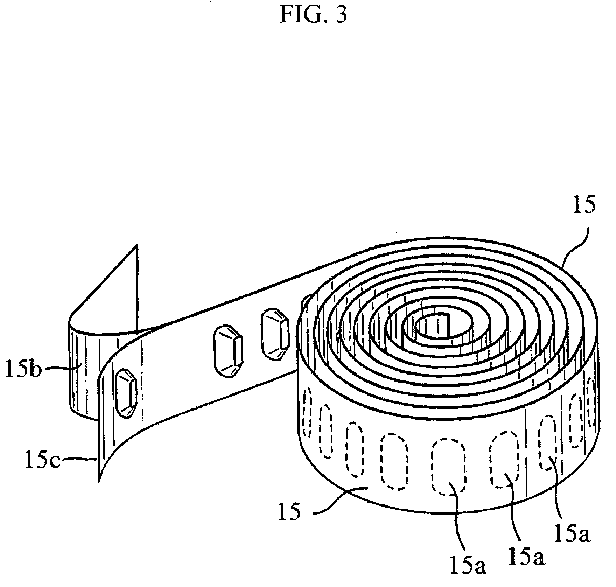

The indexing wheel (8) which engages with the indexing ratchet wheel (3) enables the blister package (15) shown in figure 3 to be indexed. The blister pockets (15a) composing the blister package are received in the recesses (8a) on the indexing wheel and the blister package (15) is indexed when the indexing wheel (8) rotates. In the inhaler pertaining to the present invention, shapes of the recesses (8a) on the indexing wheel (8) have been designed to match the shapes of the blister pockets (15) composing the blister package (15) for the blister package to be indexed properly.

The blister package (15) shown in figure 3 is composed of the lid sheet (15b) which provides impermeability and the base sheet (15c) on which the blister pockets (15a) are spaced at equal intervals. Each blister pocket contains medicament in dry powder form comprising one or more active agents.

The rotational movement that the mouthpiece cover (2) of the device executes while switching from the first position to the second is transmitted to the indexing ratchet wheel (3) via the drive gear (12) that the mouthpiece cover (2) engages with. As displayed in figure 2, arms (3a) of the indexing ratchet wheel interlocks with protrusions inside the indexing wheel (8) and rotates the indexing wheel (8) unidirectionally. Therefore, the blister package (15) is indexed forward while the indexing wheel (8a) rotates as the blister pockets (15a) composing the blister package (15) are received in the recesses (8a) of the indexing wheel. The beak (16) in the housing (10) provides the blister package (15) to be peeled while the blister package (15) is indexed and provides one blister pocket (15a) to be opened in response to each actuation of the device (1).

The winding wheel gear (6), which is another component of the gear mechanism, engages with the indexing wheel (8) as displayed in figure 2. The mechanism gear (5) that interlocks with the winding wheel (13) from inside has arms (5a) to interlock with the interior teeth of the winding wheel gear (6). When the indexing wheel (12) rotates the winding wheel gear (6), the winding wheel rotates unidirectionally owing to the arms of the mechanism gear (5a) which interlocks with the interior teeth of the winding wheel gear (6) and the lid sheet (15b) which is peeled away while the blister package is indexed is tightly coiled on the resilient wings (13a) of the winding wheel. The base sheet (15c) of the blister package (15) where the blister pockets are spaced is accumulated in a separate part (18a) of the device. Each resilient wing (13a) of the winding wheel extends from the center of the winding wheel (13) to the end.

As illustrated in figure 2a, the winding wheel (13) of the inhaler pertaining to the present invention is composed of a plurality of resilient wings which are preferably made of polyoxymethylene plastics. Each of these resilient wings (13a) is composed of three parts (A, B, C) each of which has different radius values. Thus, these resilient wings (13a) stretch enough to balance the force of attraction imposed on the lid sheet (15b) of the blister package, therefore on the blister package (15), as the thickness of the lid sheet (15b) of the blister package coiling on them increases, and they enable the blister package (15) to be indexed properly to the same extent in response to each actuation of the inhaler. The resilient wings of the winding wheel illustrated in figure 2a are composed of 3 parts (A; B; C). The average radius (R1) of the second part (B) of each resilient wing is in the range of 4,60 mm to 5,20 mm, preferably in the range of 4,75 mm to 5, 15 mm; and the radius (R2) of the piece of the third part (C) that curls through the end of the resilient wing is in the range of 0,9 mm to 1,70 mm, preferably in the range of 1,10 mm to 1,50 mm.

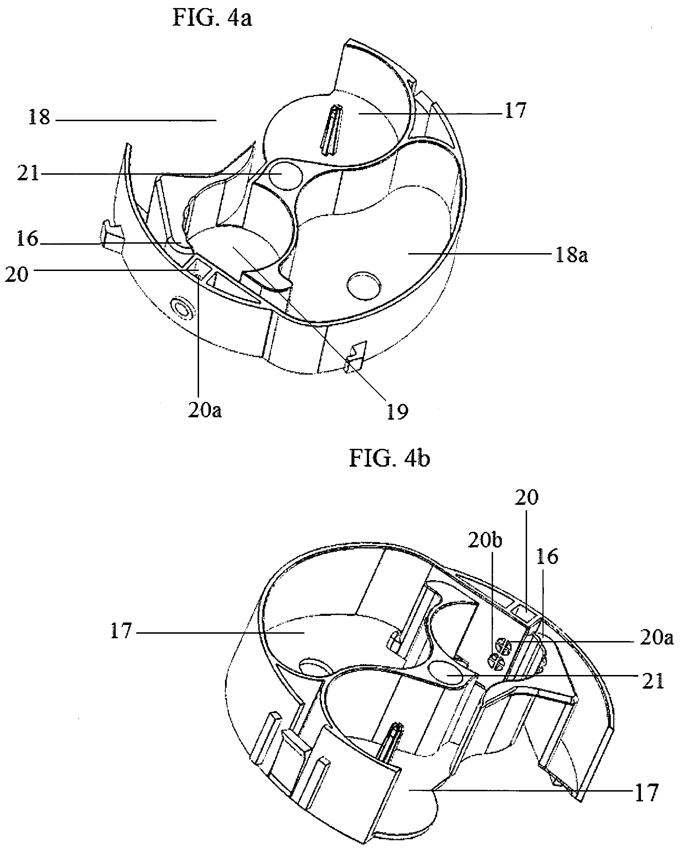

Different perspective views of the housing (10) wherein the gear mechanism and the other components of the inhaler (1) pertaining to the present invention are arranged are displayed in figures 4a and 4b. Furthermore, as can be seen in figures 4a and 4b, the housing (10) also comprises the components having significant roles in the actuation of the device such as the beak (16), the manifold (20), the apertures with four sub-apertures (20a, 20b). Each component comprised in the housing is situated in appropriate parts of the housing (10) in order to enable the inhaler (1) to work properly. The drive gear (12) passes through the center (21) of the housing and joins the mouthpiece cover (2) at both sides of the inhaler. The blister package (15) is in the lower part (17) of the housing as coiled up. In response to each actuation of the device (1), the blister package (15) is peeled by the beak (16) in the housing while being indexed by the indexing wheel (8) situated in the upper part (19) of the housing. The lid sheet (15b) of the blister package (15) which provides impermeability is indexed over the beak (16) and coiled on the winding wheel (13) which is situated in the side part (18) of the housing. The base sheet (15c) of the blister package (15) on which the blister pockets (15a) are spaced, on the other hand, is accumulated in the separated compartment (18a) of the housing (10). Upon the inhalation of the patient, the air passes through the air inlet with four sub-apertures (20a) under the manifold (20) into the opened blister pocket; entrains the dry powder medicament contained in the opened blister pocket (15a) in response to each actuation of the device; provides it to pass through the other aperture with four-sub-apertures (20b) and reach the mouthpiece via the manifold (20).

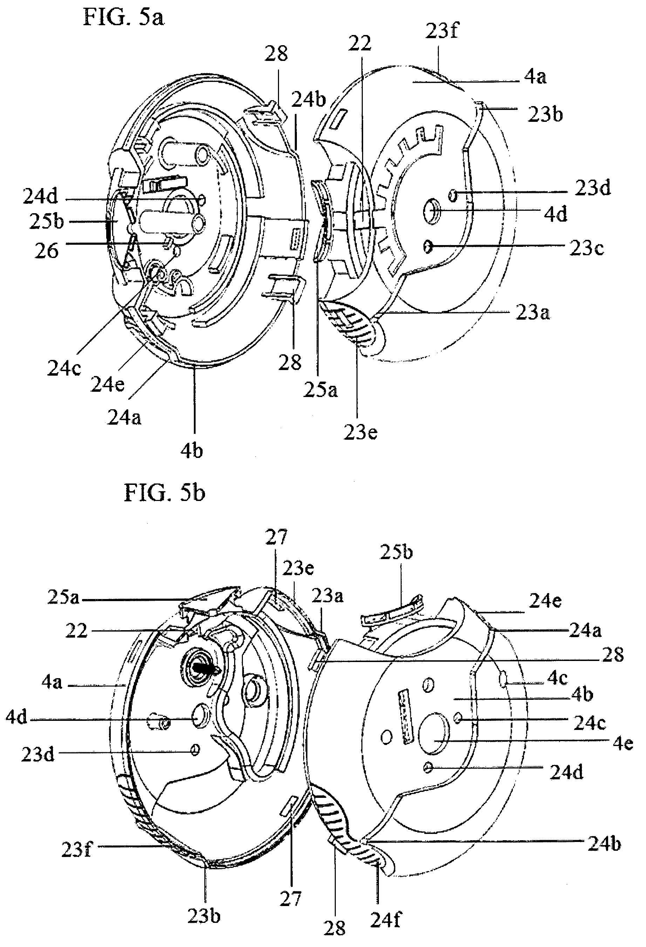

The housing (10) and the other components of the inhaler (1) pertaining to the present invention are stably kept together as the upper housing member (4a) and the lower housing member (4b) displayed in figures 5a and 5b are joined together. The engagement tabs (28) on the inside surface of the lower housing member (4b) engage with the engagement recesses (27) on the inside surface of the upper housing member (4a) and the upper and lower housing members are fixed tightly. Therefore, the protrusions (23a, 23b) on the upper housing member (4a) and the protrusions (24a, 24b) on the lower housing member (4b) are joined end to end and they define the restricted path for the rotational movement of the mouthpiece cover (2). The mouthpiece cover (2) can be moved along this path. When the mouthpiece cover (2) is on the first position, the mouthpiece is completely covered, the device is in standby mode and the mouthpiece cover (2) leans on the first protrusion (23a) on the upper housing member and the first protrusion (24a) on the lower housing member. The mouthpiece (2) is manually slid along the rotational path with the help of the carved part to switch to the second position. The mouthpiece is completely exposed when the cover is in this position, one dose of the dry powder medicament is ready for inhalation and the mouthpiece cover (2) leans on the second protrusion (23b) on the upper housing member and the second protrusion (24b) on the lower housing member.

As displayed in figures 5a and 5b, one half (25a) of the tapered channel that interconnects the manifold (20) that exist in the housing (10) with the mouthpiece (14) is comprised in the upper housing member (4a) while the other half of it (25b) is comprised in the lower housing member (4b). The channel is constituted as a whole when the upper (4a) and the lower (4b) housing members are joined together. Upon the inhalation of the patient, the air that enters the device through the air inlet (22) arranged in the upper housing member (4a) passes through the aperture with four sub-apertures (20a), reaches the opened blister (15a) and entrains the dry powder medicament there to the manifold (20) by passing it through the other aperture with four sub-apertures (20b). The grids on the upper housing member (23e, 23f) and the grids on the lower housing member (24e, 24f) prevent the slips of fingers when rotating the mouthpiece cover.

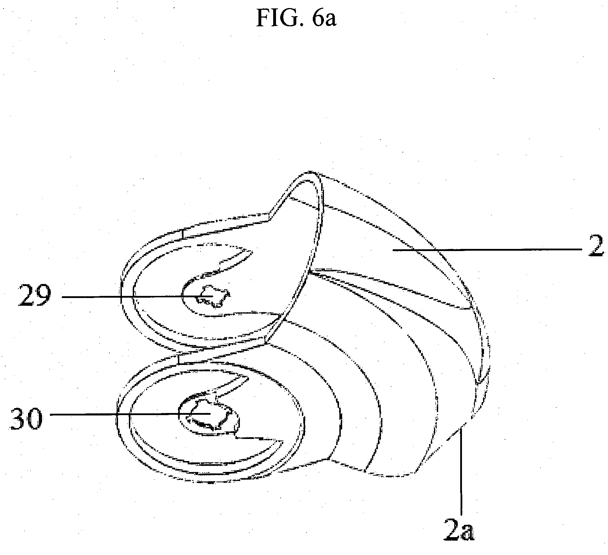

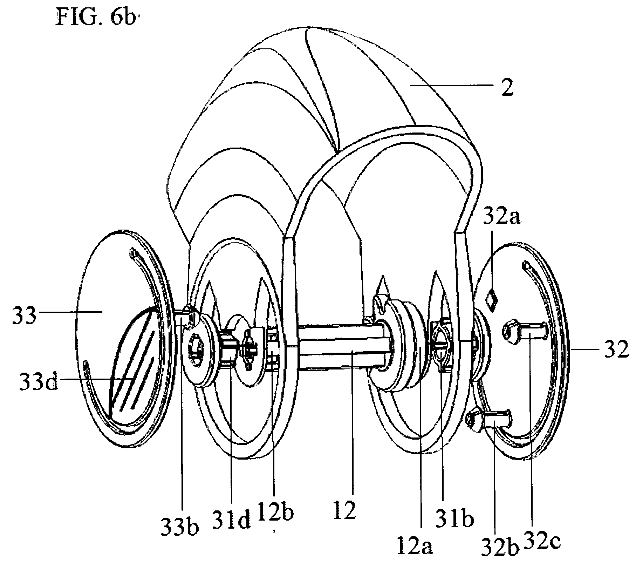

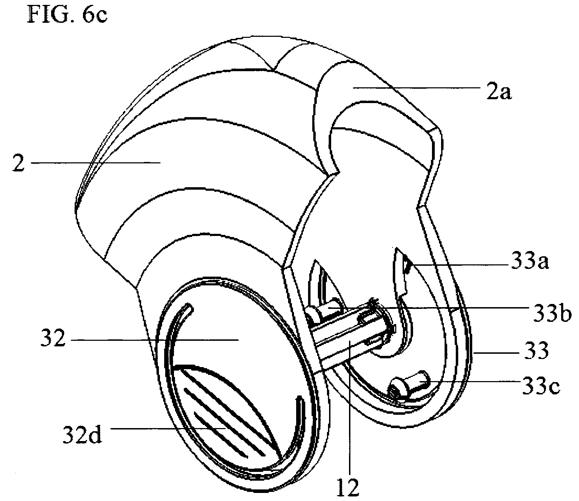

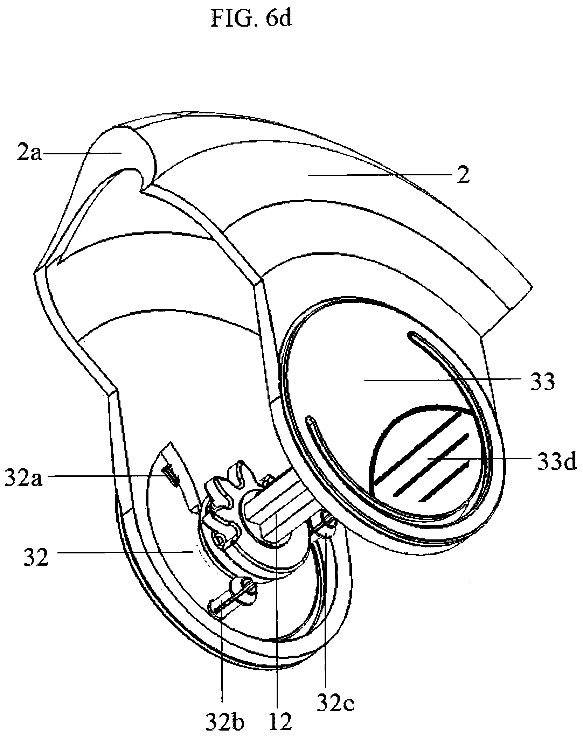

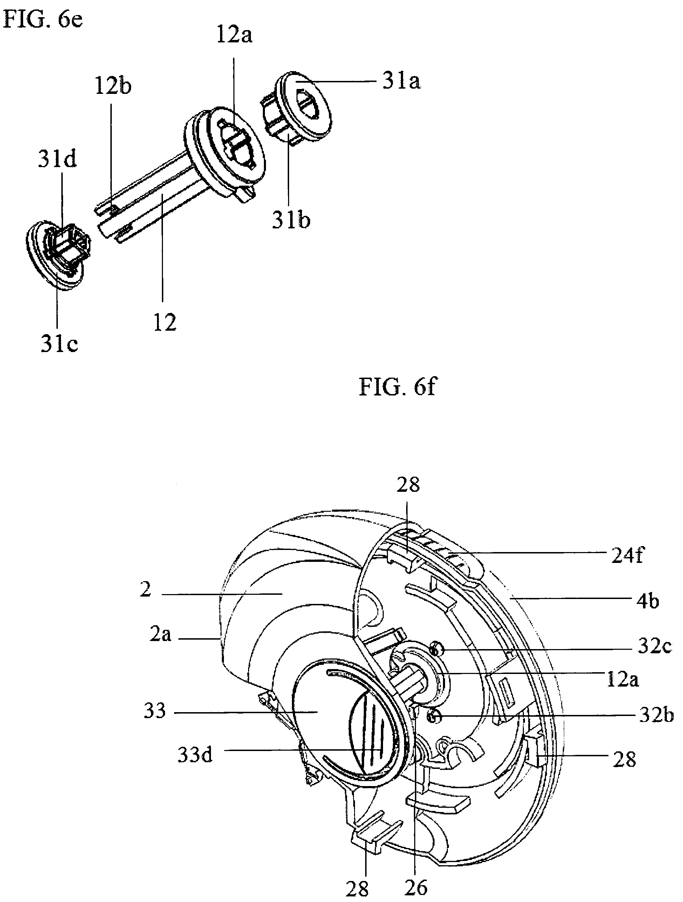

The mouthpiece cover (2) of the inhaler pertaining to the present invention is displayed in figure 6a. The carved part (2a) in one end of the device enables to easily move the mouthpiece cover manually. The mouthpiece cover (2) is joined with the gear mechanism via the connection points. The drive gear (12) is joined with the connection points (29, 30) of the mouthpiece cover via the side covers (31 a, 31 c) as it can clearly be seen in figures 6b, 6c and 6d illustrating the communication between the mouthpiece cover (2), the drive gear (12), side covers (31a, 31c) and the stabilizing resilient covers (32,33). Each of these side covers (31a; 31c) passes through the center (4d) of the upper housing member or the center (4e) of the lower housing member and joins with the end (12a; 12b) of the drive gear. It can clearly be seen in figure 6d that the both ends (12a; 12b) of the drive gear is carved such that the end of the side cover (31b; 31d) can pass through. Each end of the side covers (31d; 31b) passes through one of the connection points (29; 30) of the mouthpiece cover and it is received in the recess in one end (12b; 12a) of the drive gear, thus it provides to tightly and stably interconnect the mouthpiece cover (2) with the drive gear (12). It is provided that the mouthpiece cover (2) synchronizes with the drive gear (12) as the connection point (29; 30) of the mouthpiece cover which has a matching shape with the ends (31 d; 31 b) of the side covers that passes through it on both sides of the device and the end (12b; 12d) of the drive gear that it communicates with are on the same component.

As is seen from figures 6a-6e, the shapes of the ends (31b; 31d) of the side covers that are received in the carved parts on the ends of the drive gear and the shapes of the connection points (29, 30) of the mouthpiece cover are not identical since the two ends (12a, 12b) of the drive gear are not identical.

There is one stabilizing resilient cover (33; 32) on each connection point (29; 30) of the mouthpiece and on each side cover (31c; 31a), as displayed in figures 2, 6a-6d and 6f. When the mouthpiece cover (2) is in the first position, the pawls (32a, 33a) under the stabilizing resilient covers, which are on the connection points (29, 30) of the mouthpiece, interlock with the mouthpiece cover (2) on both sides as clearly seen in figures 6c and 6d. The pawl (33a) under the stabilizing resilient cover that is on the first connection point (29) interlocks with the mouthpiece cover on one side (figure 6c). Identically, the pawl (32a) under the stabilizing resilient cover that is on the second connection point (30) of the mouthpiece cover interlocks with the mouthpiece cover (2) on the other side (figure 6d). Thus, these pawls (32a, 33a) under the stabilizing resilient covers prevent the movement of the mouthpiece cover (2) by interlocking with it on both sides.

The extensions (32b, 32c; 33b, 33c) under the stabilizing resilient covers pass through the apertures (23c, 23d; 24c, 24d) on the upper and the lower housing members illustrated in figures 5a and 5b and provide the stabilizing resilient covers to remain stable. Namely, the extensions (33b; 33c) under the stabilizing resilient cover that is on the first connection point (29) of the mouthpiece cover pass through the apertures (23c; 23d) on the upper housing member and provide the stabilizing resilient cover (33) to be stably joined with the device. Identically, the extensions (32b, 32c) under the stabilizing resilient cover on the second connection point (30) of the mouthpiece cover pass through the apertures (24c, 24d) on the lower housing member and provide the stabilizing resilient cover (32) to be stably joined with the device as clearly illustrated in figure 6f.

Before the inhalation, the resilient parts (32d, 33d) of each stabilizing resilient cover illustrated in figures 6c and 6d are pressed on for raising the pawls (32a, 33a) and releasing the mouthpiece cover (2) in order to actuate the gear mechanism of the device to prepare one dose of dry powder medicament before inhalation. Therefore, the gear mechanism of the device is actuated and one blister pocket (15a) is opened for one dose of the dry powder medicament to be ready for inhalation when the resilient parts (32d, 33d) of the stabilizing resilient covers are pressed on and the mouthpiece cover (2) is switched from the first position to the second position simultaneously. The necessity to press on the resilient parts (32d, 33d) of the stabilizing resilient covers so as to actuate the gear mechanism preclude the consequences which may result from accidental and inadvertent actuations of the gear mechanism.

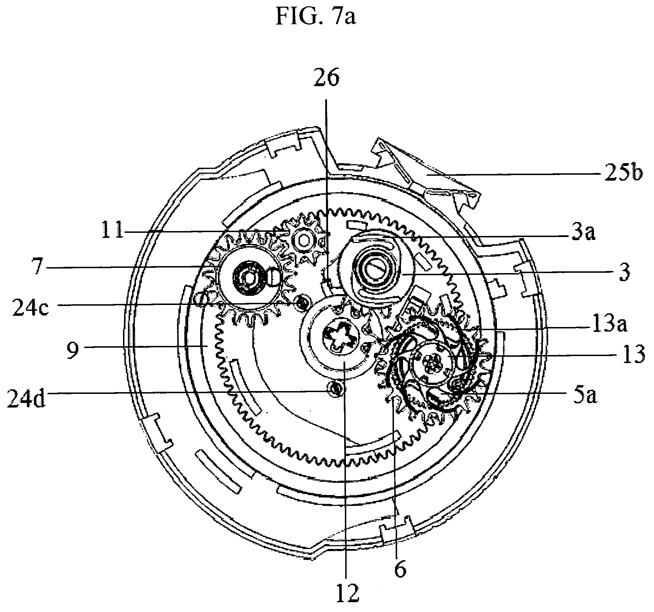

In figure 7a, it is displayed that the stopper (26) interlocks with the tooth of the indexing ratchet wheel (3) and hinders its rotation. The rotational movement of the mouthpiece cover (2) by the same angle in response to each actuation of the device (1) is accurately transmitted to the indexing ratchet wheel (3) by the drive gear (12) which engages with the mouthpiece cover (2) on its both ends and the drive gear (12) is enabled to rotate by the same angle in each actuation of the device (1). The stopper component (26) in the lower housing member (4b) prevents the backwards movement of the blister package (15) indexed by the indexing wheel (8) which synchronizes with the indexing ratchet wheel by keeping the position of the indexing ratchet wheel (3) fixed and provides the blister package (15) to be precisely positioned.

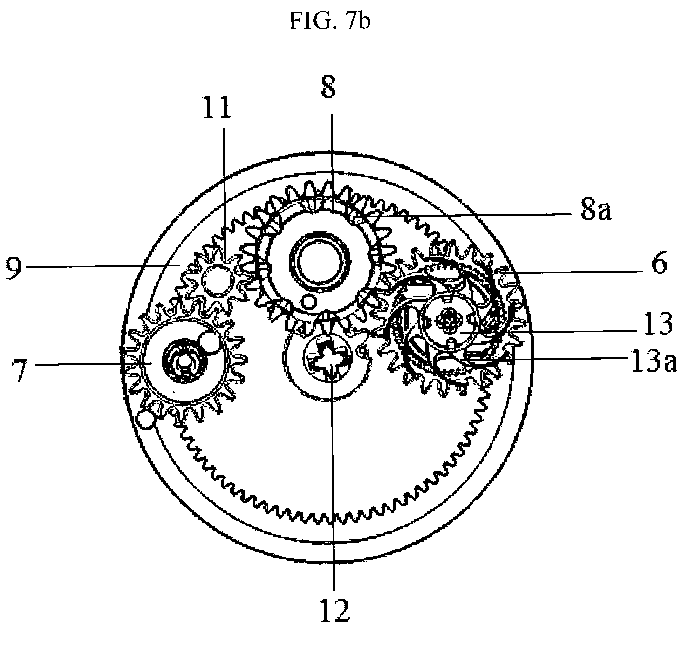

As can be seen in figure 7b, the indexing wheel (8) which synchronizes with the indexing ratchet wheel (3) is engaged with the winding wheel gear (6) and the pinion gear (11) and the rotation of the indexing wheel (8) causes the pinion gear (11) and the winding wheel gear (6) to rotate. Thus, both the peeled lid sheet (15b) of the blister package (15) which is indexed by the rotation of the indexing wheel (8) is tightly coiled on the winding wheel (13) engaging with the winding wheel gear (6) and also the counter wheel (9) is provided to be moved by the pinion gear (11) and the base gear (7) as a result of the rotation of the indexing wheel (8).

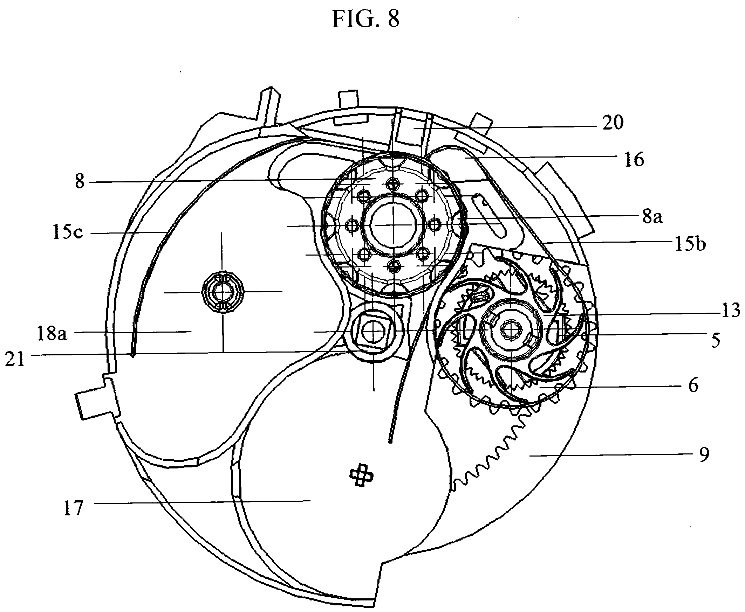

As is seen in figure 8, the lid sheet (15b) of the blister package (15) which is peeled away by the beak (16) and the base sheet (15c) are enclosed in separate compartments. The lid sheet (15b) that provides impermeability is indexed over the beak (16) and tightly coiled on the resilient wings (13a) of the winding wheel. The base sheet (15c) of the blister package (15) where the blister pockets (15a) each of which carries one dose of the dry powder medicament are spaced is accumulated in the separated compartment (18a) of the housing (10). In response to each actuation of the device (1), one dose of the dry powder medicament which is prepared for inhalation after one blister pocket (15a) is opened and the air entering the device through the air inlet (22) upon the inhalation of the patient provides to deliver one dose of the dry powder medicament to the patient by entraining it from the blister pocket (15a) to the mouthpiece (14).

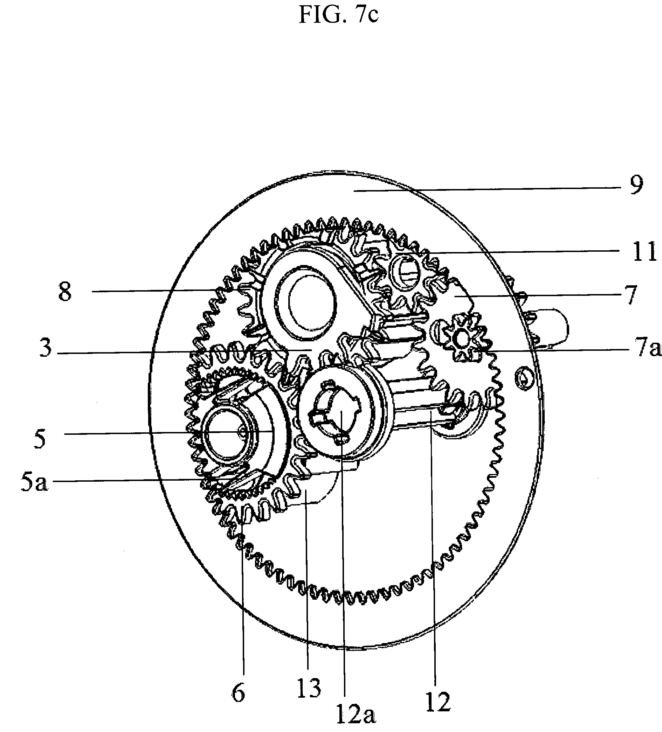

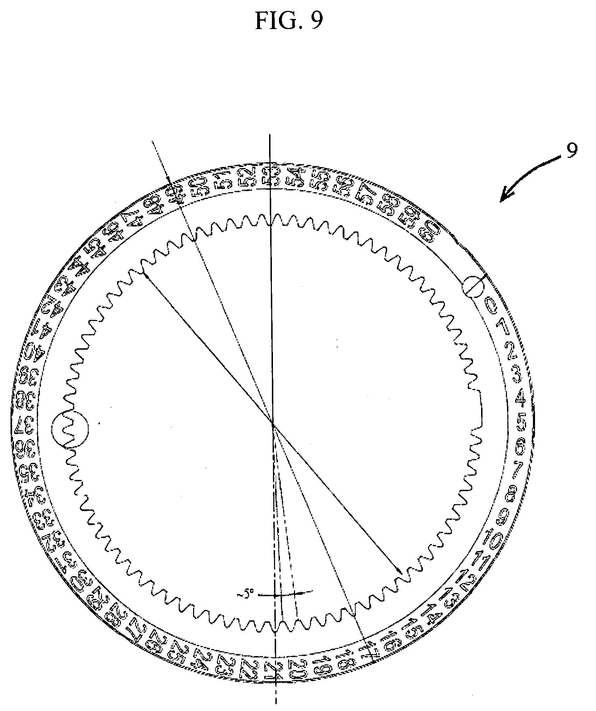

The rotation of the indexing wheel (8) is transmitted to the base gear (7) engaging with the pinion gear (11) by the pinion gear (11). The small gear which is under the base gear (7) as attached engages with the counter gear (9) (figure 7c). Thus, the movement of the indexing wheel (8) is transmitted to the counter wheel (9) shown in figure 9 by the pinion gear (11) and the base gear. There are numerals incrementing from 1 to 60 in the counter gear (9) displayed in figure 13. The angles between these numerals are all equal and approximately 5°. In response to each actuation of the device, the counter gear rotates approximately 5° and the number of the unused blister pockets remained in the device are clearly seen through the display aperture (4c) on the lower housing member (4b).

In use of the device described in figures 1-9, the mouthpiece (14) is exposed when the mouthpiece cover (2) is slid from the first position to the second on the upper housing member (4a) and the lower housing member (4b); the gear mechanism is triggered by the drive gear (12) and one dose of dry powder medicament is prepared for inhalation; the counter gear (9) is indexed and the numeral seen through the display aperture (4c) on the lower housing member (4b) is incremented. After the inhalation is realized, the mouthpiece cover (2) is solely moved from the second position to the first position wherein the mouthpiece (14) is completely covered.

The medicament in dry powder form which is stored in blister cavities is manufactured according to the prior art. According to the present invention, the particle sizes of the active agents comprised in the dry powder medicament are smaller than 20 µm, preferably smaller than 10 µm.

The inhaler pertaining to the present invention has been designed so as to deliver the dry powder medicament used in monotherapy or combined therapy. The term "monotherapy" refers to inhalation treatments in which dry powder medicaments comprising a single active agent are used whereas the term "combined therapy" refers to inhalation treatments in which dry powder medicaments comprising more than one active agents are use used.

The dry powder medicament delivered via the device pertaining to the present invention comprises at least one excipient in addition to the active agent or agents. These excipients are generally chosen from a group comprising monosaccharides (glucose, arabinose, etc.), disaccharides (lactose, saccharose, maltose, etc.), oligo- and polysaccharides (dextran, etc.), polyalcohols (sorbite, mannite, xylite), salts (sodium chloride, calcium carbonate, etc.) or combinations thereof. According to the present invention, the medicament in dry powder form comprises lactose as the excipient. The medicament in dry powder form comprises fine or coarse excipients particles preferably having various particle size ranges in order to deliver the required amount to the lungs.

The active agent or the active agents comprised in the dry powder medicament which is stored in blister packages used in the device pertaining to the present invention can be selected from a group comprising cromolyns, anti-infectives, antihistamines, steroids, anti-inflammatories, bronchodilators, leukotirene inhibitors, PDE IV inhibitors, antitussives, diuretics, anticholinergics, hormones, xanthines and pharmaceutically acceptable combinations thereof.

The active agent comprised in the medicament in dry powder form delivered via the inhaler pertaining to the present invention is preferably selected from a group comprising tiotropium, oxitropium, flutropium, ipratropium, glicopironium, flunisolid, beclomethasone, budesonide, fluticasone, mometasone, ciclesonide, rofleponide, dexamethasone, montelukast, methylcyclopropane acetic acid, sodium cromoglicat, nedocromil sodium, Npropylene, teophylline, roflumilast, ariflo (cilomilast), salmeterol, salbutamol, formoterol, terbutaline, carmoterol, indacaterol, cetirizine, levocetirizine, efletirizine, fexofenadine and their racemates, free base, enantiomers or diastereomers and their pharmaceutically acceptable salts, solvates and/or hydrates or a combination of said active agents.

The device pertaining to the present invention is used in the administration of the medicament in dry powder form which is utilized in the treatment of respiratory diseases, particularly in asthma, chronic obstructive pulmonary disorder (COPD) and allergic rhinitis. Accordingly, the respiratory diseases include, but not restricted to, allergic or non-allergic asthma at any phases, acute lung injury (ALI), acute respiratory distress syndrome (ARDS), exacerbation of airways hyperactivity, bronchiectasis, chronic obstructive pulmonary including emphysema and chronic bronchitis, airways or lung diseases (COPD, COAD or COLD), pneumoconiosis, aluminosis, anthracosis, asbestosis, chalicosis, ptilosis, siderosis, silicosis, tabacosis and byssinosis. The device pertaining to the invention can be used in prophylactic or symptomatic treatment. In addition, the medicament in dry powder form which is preferably used in the symptomatic treatment of allergic asthma and COPD is administered to the patient via the device pertaining to the present invention.

## Claims

1. An inhaler (1) suitable for delivery of the medicament in dry powder form from a blister package (15) composed of a plurality of blister pockets (15a) each of which comprises medicament in dry powder form and which are spaced at equal intervals; said inhaler comprising:
- a mouthpiece (14) enabling the patient to inhale the medicament in dry powder form from the opened blister pocket (15a),
- a gear mechanism enabling the blister package (15) to be indexed and the medicament in dry powder form to become ready for inhalation,
- a winding wheel (13) which is a member of the gear mechanism and on which the lid sheet (15b) of the blister pack is coiled, wherein each of resilient wings (13a) of the winding wheel (13) extending from the center to the end of the winding wheel on which the lid sheet (15b) of the blister package that is indexed and peeled upon the actuation of the inhaler is coiled is comprised of 3 parts (A, B, C); and the average radius (R1) of the second one (B) of these parts (A, B, C) of each resilient wings (13a) concatenating from the center to the end of the winding wheel (13) is in the range of 4,60 mm to 5,20 mm; and the radius (R2) of the piece of the third part (C) that curls through the end of the resilient wing is in the range of 0,9 mm to 1,70 mm,
- a rotatable mouthpiece cover (2) covering the mouthpiece;
- a housing (10) situated between the upper housing member (4a) and the lower housing member (4b) in which the blister package and the gear mechanism are enclosed **characterized in that** the mouthpiece cover (2) can solely be in two positions:
the mouthpiece (14) is completely covered and the device (1) is on standby mode when the mouthpiece cover (2) is in the first position,
one dose of the medicament in dry powder form is ready for inhalation upon the actuation of the device (1) when the mouthpiece cover (2) is in the second position.

2. The inhaler according to claim 1, wherein the average radius (R1) of the second part (B) of the 3 parts (A, B, C) composing each of the resilient wings (13a) extending from the center to the end of the winding wheel (13) is in the range of 4, 75 mm to 5,1 mm and the radius (R2) of the piece of the third part (C) that curls through the end of the resilient wing is in the range of 1,10 mm to 1,50 mm.

3. The inhaler (1) according to claim 1, wherein each resilient wing (13a) of the winding wheel is made of polyoxymethylene plastics.

4. The inhaler (1) according to claim 1, wherein a restricted path is arranged with the protrusions (23a, 23b; 24a, 24b) on the upper and lower housing members.

5. The inhaler (1) according to claim 1, wherein the mouthpiece cover (2) is rotationally moved by being slid on the upper (4a) and the lower (4b) housing member.

6. The inhaler (1) according to claim 1, wherein there is a carved part (2a) in one end of the mouthpiece cover so as to rotate the mouthpiece cover easily.

7. The inhaler (1) according to claim 1, wherein all components of the gear mechanism is directly or indirectly engages with each other.

8. The inhaler (1) according to either one of claim 1 or 7, wherein said gear mechanism is composed of;
- the drive gear (12) which provides to actuate the device (1) by transmitting the constant-angle movement of the mouthpiece cover (2) to the indexing ratchet wheel (3);
- the indexing wheel (8) which synchronizes with the indexing ratchet wheel (3) and enables the blister package (15) to be indexed;
- the winding wheel gear (6) which moves the winding wheel (13) via the mechanism wheel (5) upon the rotation of the indexing wheel (8);
- the pinion gear (11) and the base gear (7) that provide to transmit the movement of the indexing wheel (8) to the counter wheel (9);
- the counter wheel (9) which displays the number of the unused blister pockets (15a) remained in the device (1).

9. The inhaler according to any of the proceeding claims, further comprising a blister package (15) composed of a plurality of blister pockets (15a) each of which comprises medicament in dry powder form and which are spaced at equal intervals.

10. The inhaler (1) appropriate for delivery of the medicament in dry powder form according to any one of the preceding claims, wherein said medicament in dry powder form comprises at least one active agent selected from a group comprising cromolyns, anti-infectives, antihistamines, steroids, anti-inflammatories, bronchodilators, leukotirene inhibitors, PDE IV inhibitors, antitussives, diuretics, anticholinergics, hormones, xanthines and pharmaceutically acceptable combinations thereof.

## Patentansprüche

1. Ein Inhalator (1), der für die Abgabe des Medikaments in Form eines trockenen Pulvers aus einer Blisterverpackung (15) geeignet ist, die aus einer Vielzahl von Blistertaschen (15a) besteht, von denen jede ein Medikament in Form eines trockenen Pulvers umfasst und die in gleichen Abständen beabstandet sind; der genannte Inhalator umfasst die Folgenden;
- ein Mundstück (14), das es den Patienten ermöglicht, das Medikament in trockener Pulverform aus einer geöffneten Blistertasche (15a) im Einsatz zu inhalieren,
- ein Getriebe, das es bei Gebrauch einer Blisterverpackung (15) es ermöglicht, indiziert zu werden und, das den Medikamenten in trockener Pulverform es ermöglicht, zur Inhalation bereit zu werden,
- ein Wickelrad (13), das ein Mitglied des Getriebes ist und ,auf dem der Blechdeckel (15b) der Blisterpackung aufgewickelt wird, wobei jeder der elastischen Flügel (13a), des Wickelrades (13), die sich von der Mitte zu dem Ende des Wickelrades erstrecken, auf dem der Blechdeckel (15b) der Blisterverpackung, die bei der Betätigung des Inhalators indiziert und geschält wird, aufgewickelt wird, aus 3 Teilen (A, B, C) besteht; und der durchschnittliche Radius (R1) der zweiten (B) dieser Teile (A, B, C) jeder elastischen Flügel (13a), verbindend von der Mitte bis zum Ende des Wickelrades (13) ist im Bereich von 4,60 mm bis 5,20 mm; und der Radius (R2) des dritten Teils (C), der durch das Ende des elastischen Flügels rollt, ist im Bereich von 0,9 mm bis 1,70 mm,
- eine drehbare Mundstückabdeckung (2), die das Mundstück abdeckt;
- ein Gehäuse (10), das sich zwischen dem oberen Gehäuseelement (4a) und dem unteren Gehäuseelement (4b) befindet, in dem die Blisterpackung und das Getriebe eingeschlossen sind, **dadurch gekennzeichnet, dass** die Mundstückabdeckung (2) nur in zwei Positionen angeordnet sein kann;
das Mundstück (14) ist vollständig abgedeckt und das Gerät (1) ist im Standby-Modus, wenn sich die Mundstückabdeckung (2) in der ersten Position befindet,
eine Dosis des Medikaments in Trockenpulverform ist bei der Betätigung des Geräts (1) zur Inhalation bereit, wenn sich die Mundstückabdeckung (2) in der zweiten Position befindet.

2. Inhalator nach Anspruch 1, wobei der durchschnittliche Radius (R1) des zweiten Teils (B) der 3 Teile (A, B, C) umfassend jede der elastischen Flügel (13a), die sich von der Mitte bis zum Ende des Wickelrades (13) erstrecken, im Bereich von 4,75 mm bis 5,1 mm liegt und der Radius (R2) des Stücks des dritten Teils (C), der durch das Ende des elastischen Flügels rollt, im Bereich von 1,10 mm bis 1,50 mm liegt.

3. Inhalator (1) nach Anspruch 1, wobei jeder elastische Flügel (13a) des Wickelrades aus Polyoxymethylen-Kunststoff besteht.

4. Inhalator (1) nach Anspruch 1, wobei ein eingeschränkter Pfad mit den Vorsprüngen (23a, 23b; 24a, 24b) auf den oberen und unteren Gehäuseelementen angeordnet ist.

5. Inhalator (1) nach Anspruch 1, wobei die Mundstückabdeckung (2) sich dreht, indem sie auf den oberen (4a) und den unteren (4b) Gehäuseelementen geschoben wird.

6. Inhalator (1) nach Anspruch 1, wobei es ein geschnitzter Teil (2a) an einem Ende der Mundstückabdeckung gibt, um die Mundstückabdeckung leicht zu drehen.

7. Inhalator (1) nach Anspruch 1, wobei alle Komponente des Getriebes direkt oder indirekt in Eingriff miteinander stehen.

8. Inhalator (1) nach einem der Ansprüche 1 oder 7, wobei der genannte Getriebemechanismus die Folgenden umfasst;
- ein Antriebsrad (12), das das Gerät (1) durch Übertragen der konstanten Winkelbewegung der Mundstückabdeckung (2) an ein Indizierungssperrrad (3) auslöst;
- ein Indizierungsrad (8), das mit dem Indizierungssperrrad (3) synchronisiert wird und der Blisterverpackung (15) es ermöglicht, indiziert zu werden;
- ein Wickelzahnrad (6), das das Wickelrad (13) über den Radmechanismus (5) beim Drehen des Indizierungsrades (8) bewegt;
- ein Ritzel (11) und Basiszahnrad (7), die die Bewegung des Indizierungsrades (8) zu einem Gegenzahnrad (9) übertragen;
- ein Gegenzahnrad (9), das die Anzahl der nicht verwendeten und im Gerät (1) verbleibender Blistertaschen (15a) zeigt.

9. Inhalator nach einem der vorstehenden Ansprüche ferner umfassend eine Blisterpackung (15), die aus einer Vielzahl von Blistertaschen (15a) besteht, von denen jede ein Medikament in Form eines trockenen Pulvers umfasst und die in gleichen Abständen beabstandet sind.

10. Inhalator (1) geeignet für die Abgabe des Medikaments in Trockenpulverform nach einem der vorhergehenden Ansprüche, wobei das genannte Medikament in Form eines trockenen Pulvers wenigstens einen Wirkstoff beinhaltet, der aus einer Gruppe umfassend cromolyns, Antiinfektiva, Antihistamine, Steroide, entzündungshemmende Mittel, Bronchodilatatoren, leukotirene-Inhibitoren, PDE IV-Inhibitoren, Antitussiva, Diuretika, Anticholinergika, Hormone, Xanthine und pharmazeutisch annehmbare Kombinationen davon gewählt wird.

## Revendications

1. Un inhalateur (1) approprié pour la livraison du médicament sous la forme de poudre sèche à partir d'un emballage blister (15) composée d'une pluralité de poches blisters (15a) dont chacune comprend le médicament sous la forme de poudre sèche et qui sont espacées à des intervalles égaux ; le inhalateur comprenant :
- un embout (14) permettant au patient d'inhaler le médicament sous la forme de poudre sèche depuis la poche blister ouverte (15a),
- un mécanisme d'engrenage permettant l'emballage blister (15) à être indexé et le médicament sous la forme de poudre sèche à être prêt pour l'inhalation,
- une roue d'enroulement (13) qui est un membre du mécanisme d'engrenage et sur laquelle la plaque de couvercle (15b) de l'emballage blister est enroulée,
dans lequel chacune des ailes élastiques (13a) de la roue d'enroulement (13) s'allongeant du centre vers l'extrémité de la roue d'enroulement sur laquelle la plaque de couvercle (15b) de l'emballage blister qui est indexé et pelé lors de l'actionnement de l'inhalateur est enroulé est composée de 3 parties (A, B, C);
et le rayon moyen (R1) de la seconde (B) de ces parties (A, B, C) de chacune des ailes élastiques (13a) enchaînant du centre vers l'extrémité de la roue d'enroulement (13) est dans la plage de 4,60 mm à 5,20 mm;
et le rayon (R2) de la pièce de la troisième partie (C) frisant par l'extrémité de l'aile élastique est dans la plage de 0,9 mm à 1,70 mm,
- une couverture rotative de l'embout (2) couvrant l'embout ;
- un boîtier (10) situé entre l'élément de boîtier supérieur (4a) et l'élément de boîtier inférieur (4b), dans lequel l'emballage blister et le mécanisme d'engrenage sont enfermés **caractérisé en ce que** la couverture de l'embout (2) peut seulement être dans deux positions :
l'embout (14) est complètement couvert et le dispositif (1) est en mode veille lorsque la couverture de l'embout (2) est dans la première position,
une dose du médicament sous forme d'une poudre sèche est prête pour inhalation en l'actionnement du dispositif (1) lorsque la couverture de l'embout (2) est dans la seconde position.

2. L'inhalateur selon la revendication 1, dans lequel le rayon moyen (R1) de la seconde partie (B) de ces 3 parties (A, B, C) composante chacune des ailes élastiques (13a) enchaînant du centre vers l'extrémité de la roue d'enroulement (13) est dans la plage de 4,75 mm à 5,1 mm
et le rayon (R2) de la pièce de la troisième partie (C) frisant par l'extrémité de l'aile élastique est dans la plage de 1,10 mm à 1, 50 mm.

3. L'inhalateur (1) selon la revendication 1, dans lequel chacune des ailes élastiques (13a) de la roue d'enroulement est fait d'une plastique de polyoxyméthylène.

4. L'inhalateur (1) selon la revendication 1, dans lequel une route restreinte est arrangée avec les saillies (23a, 23b; 24a, 24b) sur les éléments de boîtier supérieur et inférieur.

5. L'inhalateur (1) selon la revendication 1, dans lequel la couverture de l'embout (2) est déplacée par rotation par glissement sur l'élément de boîtier supérieur (4a) et inférieur (4b).

6. L'inhalateur (1) selon la revendication 1, dans lequel il y a une partie gravée (2a) dans une extrémité de la couverture de l'embout afin de tourner la couverture de l'embout facilement.

7. L'inhalateur (1) selon la revendication 1, dans lequel tous les composants du mécanisme d'engrenage s'engagent les uns avec les autres directement ou indirectement.

8. L'inhalateur (1) selon l'une quelconque des revendications 1 ou 7, dans lequel ledit mécanisme d'engrenage est composé de :
- l'engrenage d'entrainement (12) qui permet d'activer l'appareil (1) en transmettant le mouvement d'un angle constant de la couverture de l'embout (2) à la roue à rochet d'indexation (3) ;
- la roue d'indexation (8) qui synchronise avec la roue à rochet d'indexation (3) et permet l'emballage coque (15) à être indexé ;
- l'engrenage de roue d'enroulement (6) qui déplace la roue d'enroulement (13) par un mécanisme de roue (5) sur la rotation de la roue d'indexation (8) ;
- l'engrenage à pignons (11) et un engrenage de base (7) qui prévoit à transmettre le mouvement de la roue d'indexation (8) à la roue de contrepoussée (9) ;
- la roue de contrepoussée (9) qui affiche le nombre des poches blisters (15a) inutilisées restantes dans l'appareil (1).

9. L'inhalateur selon l'une quelconque des revendications précédentes comprenant également l'emballage blister (15) qui est composé d'une pluralité des poches blisters (15a), chacune desquelles comprenant le médicament sous la forme de poudre sèche et qui sont espacées à des intervalles égaux.

10. L'inhalateur (1) approprié pour délivrer le médicament sous forme d'un poudre sèche selon l'une quelconque des revendications précédentes, dans laquelle ledit médicament sous forme d'un poudre sèche comprend au moins un principe actif choisi d'un groupe comprenant les cromolyns, les anti-infectieux, les antihistaminiques, les stéroïdes, les anti-inflammatoires, les bronchodilatateurs, les inhibiteurs des leucotriènes, les inhibiteurs de PDE IV, les antitussifs, les diurétiques, les anticholinergiques, les hormones, les xanthines et les combinaisons pharmaceutiquement acceptables de ceux-ci.
